# EUROPÄISCHE PATENTSCHRIFT

(11) **EP 1 358 310 B1**
(45) Veröffentlichungstag und Bekanntmachung des Hinweises auf die Patenterteilung: **02.01.2008**
(21) Anmeldenummer: 02714129.0
(22) Anmeldetag: 29.01.2002
(51) Int. Cl.: C11D 17/00, C11D 17/06

(54) **WASCH- UND REINIGUNGSMITTEL UMFASSEND FEINE MIKROPARTIKEL MIT REINIGUNGSMITTELBESTANDTEILEN**
WASHING AND CLEANING AGENTS COMPRISING FINE MICROPARTICLES WITH CLEANING AGENT COMPONENTS
PRODUITS DETERGENTS ET NETTOYANTS POSSEDANT DES MICROPARTICULES FINES QUI RENFERMENT DES CONSTITUANTS DE PRODUITS NETTOYANTS

(30) Priorität: 07.02.2001 DE 10105801
(43) Veröffentlichungstag der Anmeldung: 05.11.2003
(73) Patentinhaber: Henkel Kommanditgesellschaft auf Aktien, 40589 Düsseldorf (DE)
(72) Erfinder: WEBER, Henriette, 40591 Düsseldorf (DE); RÄHSE, Wilfried, 40589 Düsseldorf (DE)
(86) Internationale Anmeldenummer: PCT/EP2002/000881
(87) Internationale Veröffentlichungsnummer: WO 2002/062937

(56) Entgegenhaltungen:
- EP-A- 0 340 989
- EP-A- 0 953 631
- EP-A- 0 987 317
- WO-A-01/00766
- WO-A-99/10467
- DE-A- 19 828 579
- DE-A- 19 855 676
- US-A- 3 418 243
- US-A- 5 904 758

## Beschreibung

Der Gegenstand der vorliegenden Anmeldung betrifft Wasch- und Reinigungsmittel, die feine Mikropartikel mit Reinigungsmittelbestandteilen aufweisen, deren Verwendung sowie ein Verfahren zu ihrer Herstellung.

Wasch- und Reinigungsmittel geeignet zur Reinigung von harten und weichen Oberflächen sind im Stand der Technik gut bekannt. Wasch- und Reinigungsmittel die Reinigungsmittelbestandteile in fester Form aufweisen, hierzu zählen beispielsweise Pulver, Tabletten und Lösungen sowie Gele die Feststoffpartikel enthalten, haben den Nachteil, das beim Lösen solcher Partikel die Gefahr einer Vergelung besteht.

So weisen Wasch- und Reinigungsmittelpartikel beim Dispergier- und Auflösungsprozeß zu Beginn der Auflösungsphase im wäßrigen Medium nach einigen Minuten Auflösungszeit eine "Korona" um das Waschmittelteilchen herum auf. Dies ist eine Gelphase, häufig mit flüssigkristallinen Phasen, die den weiteren Diffusionsprozeß und damit die Auflösungsgeschwindigkeit stark behindern kann(= Vergelungseffekt).

So tritt insbesondere bei Waschmitteln beim Lösen von Reinigungsmittelaktivstoffen, beispielsweise Tenside oder dergleichen, in der Einspülkammer oder in der Waschflotte Vergelung auf.

Die Vergelung von Reinigungsmitteiaktivstoffen führt zu einer schlechteren Dispergierbarkeit, einer verminderten Löslichkeit, einer inhomogenen Verteilung in der Waschflotte, einer verschlechterten Reinigungswirkung sowie zur Bildung von Reinigungsmittelrückständen in der Einspülkammer und/oder auf der Kleidung.

Ein weiterer Nachteil dieser im Stand der Technik bekannten Wasch- und Reinigungsmittel ist es, daß die üblicherweise in Wasch- und Reinigungsmitteln verwendeten Partikel aufgrund ihrer Größe bereits bei einer geringen Inhomogenität der Wasch- und Reinigungsmittelzusammensetzung zu deutlichen Konzentrationsunterschieden im Wasch- und Reinigungsmittel und infolge dessen auch am Verwendungsort führen. Hierdurch kann die Reinigungsleistung beeinträchtigt werden.

Um diesem Effekt entgegenzuwirken weisen die Wasch- und Reinigungsmittel häufig einen zu hohen Gehalt an Reinigungsmittelaktivstoffen, Duftstoffen, Farbstoffen und dergleichen auf.

Noch ein Nachteil der im Stand der Technik bekannten festen Waschmittel ist es, daß hohe Niotensidanteile zu klebrigen Waschmitteln führen. Es sind aber speziell die Niotenside, die insbesondere bei fettartigen Verschmutzungen, hervorragende Reinigungsleistungen besitzen. Ein hoher Anteil an Niotensiden kann, wenn die Tenside inhomogen verteilt sind und sich speziell an der Partikeloberfläche befinden zu einer mangelnden Silierbarkeit und/oder zu durch Verklumpung schlechte Schütt-, Förder- bzw. Rieselfähigkeiten führen.

Ein weiterer Nachteil bei festen Wasch- und Reinigungsmitteln ist es, daß zu große Partikel häufig ein schlechtes Auflöseverhalten aufweisen.

Schließlich werden üblicherweise bei festen Waschmitteln Duftstoffe nach Herstellung des Pulvers oder Granulats in einem nachgeschalteten Verfahrensschritt auf die Oberfläche des Produkts aufgedüst. Nachteilig bei diesem Verfahren ist es, daß der Duftstoff dadurch im Pulver bzw. Granulat nicht eingeschlossen ist, und so von der Oberfläche leicht verdunsten kann. Um über einen verlängerten Gebrauchszeitraum am Verwendungsort einen ausreichend wahrnehmbaren Duft des Wasch- und Reinigungsmittels aufrecht zu erhalten, wird deshalb der Duftstoff überdosiert um der erhöhten Verdunstung entgegenzuwirken.

Wasch- und Reinigungsmittelformkörper der Stand der Technik sind beispielsweise in den Dokumenten DE-A-198 28 579 und EP-A-1 043 391 beschrieben.

Aufgabe der vorliegenden Erfindung ist es, die vorstehend genannten Nachteile im Stand der Technik zu überwinden.

Diese Aufgabe wird erfindungsgemäß durch Wasch- und Reinigungsmittelformkörper gemäss Anspruch 1 gelöst, wobei der Wasch- und Reinigungsmittelformkörper feine Mikropartikel umfasst, wobei die feinen Mikropartikel einen oder mehrere Reinigungsmittelbestandteile aufweisen.

Weitere vorteilhafte Ausführungsformen sind in den Unteransprüchen aufgeführt, auf die im vollem Umfang Bezug genommen wird.

Es wurde nunmehr überraschend gefunden, daß bei Reinigungsmittelbestandteile enthaltenden feinen Mikropartikeln der Vergelungseffekt am Verwendungsort bei Verdünnung mit einem wässrigen Medium nicht auftritt oder zumindest deutlich verringert wird. So wurde bei Tensiden, insbesondere bei Niotensiden festgestellt, daß der Vergelungseffekt in der Einspülkammer und Wasch-flotte nicht oder nur in einem sehr geringen Umfang auftrat.

Ferner wurde für Wasch- und Reinigungsmittel, die Reinigungsmittelbestandteile in Form feiner Mikropartikel umfassen, eine bessere Dispergierbarkeit, eine gesteigerte Lösungsgeschwindigkeit, eine homogenere Verteilung und eine verbesserte Reinigungswirkung in der Waschflotte festgestellt.

Außerdem werden Verkrustungen, beispielsweise verursacht durch mittels Vergelung gebildeter Klumpen oder dergleichen, in der Einspülkammer, in Zu- und Ableitungen durch Waschmittelrückstände verhindert oder zumindest reduziert.

Diese Wirkungen sind vermutlich, ohne auf eine bestimmte Theorie festgelegt zu sein, insbesondere auf den verminderten Vergelungseffekt zurückzuführen.

Ein weiterer Vorteil von Reinigungsmittelbestandteilen in Form feiner Mikropartikel ist, daß diese in Wasch- und Reinigungsmitteln eine gleichmäßigere bzw. homogenere Verteilung erlauben.

Außerdem läßt sich durch eine gleichmäßige Verteilung der erfindungsgemäßen Reinigungsmittelbestandteile in Form feiner Mikropartikel, beispielsweise für Tenside, insbesondere Niotenside, bedingt durch die Stützwirkung der umgebenden Pulvermatrix des Wasch- oder Reinigungsmittels, ein höherer Gewichtsanteil, bezogen auf die Wasch- und Reinigungsmittelgesamtzusammensetzung, erreichen. Die bessere Verteilung des Niotensids oder des Parfüms vermindert hierbei die Neigung zur Klebrigkeit des Gesamtproduktes. Aufgrund des verminderten Vergelungseffekts kann die Reinigungsleistung des Wasch- und Reinigungsmittels gesteigert werden, da mehr Wasch- und Reinigungsmittelpartikelbestandteile zu Beginn des Waschprozesses in Lösung gehen und so am Reinigungsprozess aktiv beteiligt sind.

Die Verteilung der Wasch- und Reinigungsmittelbestandteile, wie Tenside, Duftstoffe oder dergleichen, in einem feinen Mikropartikel erfolgt vorteilhafterweise durch ein Carrier-Prinzip. Beim Carrier-Prinzip muß der Duftstoff in das feine Mikropartikel hineingetragen werden, ohne es zu zerstören. Nach dem Eintragen muß das Wasch- und Reinigungsmittelbestandteil im Partikel festgehalten werden, ohne daß bei Lagerung eine Entmischung, beispielsweise Drängen an die Partikeloberfläche o.ä., stattfindet.

Da Duftstoffe wie Parfümöle hydrophob sind, führen größere Parfümölgehalte, z.B. bei Duftperlen zur Beimischung in Waschmittelzubereitungen, ebenfalls zu Dispergier- und Löslichkeitsproblemen. Feine Mikropartikel, die Duftstoff- bzw. Parfümgehalte aufweisen, führen, wie bei den Niotensiden, somit auch zu einem besseren Einspül- und Dispergierverhalten als grobe Partikel mit den gleichen Inhaltsstoffen.

Wichtig ist, daß die feinteiligen Duftstoffe, wie Parfüm- oder Tensidpartikel etc, in der gebrauchsfertigen Wasch- und Reinigungsmittelzusammensetzung unzerstört enthalten sind. Werden die feinen Mikropartikel zerdrückt und/oder mit der Wasch- und Reinigungsmittel-Restmatrix vermengt, treten in Folge des Geleffekts ungünstigere Dispergier-, Löse- und Lagereigenschaften auf.

Die Verwendung von feinteiligen Duftstoff- und Tensidpartikeln in Wasch- und Reinigungsmittelzubereitungen, insbesondere von Tabs, Granulaten und/oder Extrudaten, ist besonders vorteilhaft.

Die erfindungsgemäßen Wasch- und Reinigungsmittel umfassen wenigstens einen formkörper. Es handelt sich bei Formkörper(n) um verdichtete Formkörper. Die Formkörper aufweisen feine Mikropartikel. Die feinen Mikropartikel können auf der äußeren Oberfläche des Formkörpers angebracht sein, den Formkörper als solches bilden und/oder in dem Formkörper enthalten sein.

Besonders bevorzugt ist, daß die Formkörper am Verwendungsort Mikropartikel freisetzen. Verwendungsort im Sinne dieser Erfindung ist der Ort und der Zeitpunkt zu dem die Wasch- und Reinigungsmittelbestandteile zwecks Reinigung bzw. Schmutzentfernung freigesetzt werden und/oder die Wasch- und Reinigungsmittel zwecks Reinigung bzw. Schmutzentfernung aktiv werden. Die Freisetzung der Mikropartikel erfolgt vorzugsweise zu Beginn des Wasch-/Reinigungsprozesses, kann aber auch zu einem früheren oder späteren Zeitpunkt erfolgen. Der Zweck der Freisetzung kann auch eine Gewebekonditionierung oder eine Parfümierung sein.

Der Kompaktierungsgrad der Formköper ist vorzugsweise so gewählt, daß der Formkörper am Verwendungsort feine Mikropartikel freisetzt. Die Mikropartikel besitzen ein größeres Verhältnis von Oberfläche zu Volumen als grobe Partikel und verteilen sich besser im Lösungsmedium. Auf diese Weise wird bei erfindungsgemäßen Waschmitteln, wenn sich die Formkörper in der Waschflotte lösen, durch die Freisetzung feiner Mikropartikel dem Vergelungseffekt entgegengewirkt.

Geeignete Formkörper umfassen Granulate, Extrudate, Agglomerate, und Tabletten, sowie Mischungen davon.

Diese Formkörper können auch in einer umgebenden flüssigen Waschmittel- oder Reinigerumgebung eingesetzt werden. Ferner können die feinen Mikropartikel in dem Wasch- und Reinigungsmittel in kompaktierter und/oder nicht kompaktierter Form vorliegen.

Erfindungsgemäß besonders bevorzugt ist es, daß die feinen Mikropartikel in Wasch- und Reinigungsmittelzusammensetzung zumindest teilweise unzerstört, vorzugsweise unzerstört enthalten sind.

Bevorzugt sind solche Agglomerate, Tabletten, wie Tabs und/oder dergleichen, sowie Mischungen davon, die am Einsatzort, beispielsweise in der Waschflotte die feinen Mikropartikel wieder freisetzten. Die feinen Mikropartikel können aber auch mit anderen Waschmittelbestandteilen zu Agglomeraten kompaktiert werden. Bevorzugt sind insbesondere sogenannte Megaperls, erhältlich von der Henkel KGaA. Bevorzugt sind solche Megaperls, die die feinen Mikropartikel am Einsatzort, beispielsweise in der Waschflotte, wieder freisetzten.

Besonders vorteilhaft ist es, daß man durch Verwendung der feinen Mikropartikel Wasch- und Reinigungsmittelagglomerate bilden kann, wie beispielsweise Megaperls, in denen mittels der Beimischung von feinen Mikropartikeln eine feinteilige, d.h. sehr gute Volumenverteilung der feinen Mikropartikel, und gleichzeitig eine gute homogene Verteilung erreicht werden kann.

Es hat sich bei dem erfindungsgemäßen Wasch- und Reinigungsmittel gezeigt, daß insbesondere bei Waschmitteln, Granulaten, Extrudaten und Wasch- und Reinigungsmitteltabletten, durch Verwendung von Duftstoffe aufweisenden feinen Mikropartikeln, ein Teil des Duftstoffs bereits beim Öffnen der Verpackung verduftet, wodurch ein guter Dufteindruck beim Verbraucher entsteht, aufgrund der Duftstoffe enthaltenden feinen Mikropartikel aber eine ausreichende Menge an Duftstoff im Wasch- und Reinigungsmittel verbleibt, so daß über einen verlängerten Verbrauchszeitraum, insbesondere bis zum Aufbrauch des Wasch- und Reinigungsmittels der gute Dufteindruck erhalten bleibt. Außerdem können auf diese Weise in der Waschflotte ebenfalls aureichende Duftstoffmengen freigesetzt werden.

Der Duftstoffanteil in Wasch- und Reinigungsmitteln, insbesondere bei Waschmittelfestkörpern ist sehr gering. Üblicherweise beträgt der Gehalt 0,5 Gew.-% bezogen auf die Gesamtzusammensetzung des Wasch- und Reinigungsmittel. Um eine homogene Verteilung der Duftstoffe zu gewährleisten ist eine sehr feinteilige Verteilung, die durch die feinen Mikropartikel gewährleistet werden kann, sehr vorteilhaft. Dies gilt sowohl für das Wasch- und Reinigungsmittel als solches, beispielsweise Waschmittelpulver, als auch für Wasch- und Reinigungsmittelagglomerate bzw. Waschmittelpartikel wie Megaperls, bei denen eine gleichmäßige, feinteilige Verteilung wünschenswert ist.

Herkömmliche Megaperls weisen einen Durchmesser von 1,4 mm auf und wiegen 0,1 g, d.h. bei gleichmäßiger Duftstoffverteilung über alle Megaperls und 0,5% Duftstoff ergibt sich eine Menge von 0,5 mg Duftstoff. Als einziger Tropfen ist dies ein Tropfen mit einem Durchmesser von ca. 200 µm. Um eine möglichst optimale, gleichmäßige Verteilung des Duftstoffes innerhalb des Megaperls und an deren Oberfläche zu erreichen sind feine Mikropartikel ≤ 20 µm besonders vorteilhaft.

Der Begriff "feine Mikropartikel" im Sinne dieser Erfindung umfasst unter anderem auch sehr kleine Partikel, wie Nanopartikel.

Die Korngrößen der feinen Mikropartikel sind ≤ 100 µm, bevorzugt ≤ 50 µm und weiter bevorzugt ≤ 20 µm. Die Korngrößen der feinen Mikropartikel können aber auch ≤ 10 µm, bevorzugt ≤ 5 µm, weiter bevorzugt ≤ 2 µm, ≤ 1 µm, ≤ 0,5 µm, ≤ 0,1 µm, ≤ 0,05 µm und sogar ≤ 0,01 µm sein.

Geeignete Korngrößen der feinen Mikropartikel sind x₉₀=80 µm und besonders bevorzugt x₉₀=5 µm.

In einer bevorzugten Ausführungsform der Erfindung weisen die feinen Mikropartikel der Wasch-und Reinigungsmittel eine Korngröße von zwischen 0,001 µm bis 50 µm, vorzugsweise von zwischen 0,01 µm bis 20 µm, bevorzugt von zwischen 0,05 µm bis 10 µm, weiter bevorzugt von zwischen 0,1 µm bis 5 µm, und am meisten bevorzugt von zwischen 0, 5 µm bis 1 µm, auf.

In einer weiteren bevorzugten Ausführungsform der Erfindung weisen ≥ 10%, vorzugsweise ≥ 30%, bevorzugt ≥ 50%, weiter bevorzugt ≥ 70%, noch bevorzugter ≥ 80%, und am meisten bevorzugt ≥ 90% der feinen Mikropartikel, bezogen auf die Gesamtanzahl der feinen Mikropartikel, eine Korngrößenverteilung von ≤ 20 µm auf.

Besonders vorteilhaft ist es, wenn die feinen Mikropartikel einem mechanischen Verdichtungsprozeß unterworfen werden.

Beispielsweise kann das Wasch- und Reinigungsmittel feine Mikropartikel, die einem mechanischen Verdichtungsprozeß unterworfen wurden, aufweisen, wobei diese feinen Mikropartikel im Wasch- und/oder Reinigungsprozeß unzerstört freigesetzt werden.

Außerdem aufweist das Wasch- und Reinigungsmittel Formkörper, die einem mechanischen Verdichtungsprozeß unterworfen wurden, wobei die Formkörper unzerstörte Mikropartikel aufweisen können.

In einer bevorzugten Ausführungsform weisen die Wasch- und Reinigungsmittel feine Mikropartikel auf, die einem mechanischen Verdichtungsprozeß unterworfen wurden, wobei ≥ 10%, vorzugsweise ≥ 30%, bevorzugt ≥ 50%, weiter bevorzugt ≥ 70%, noch bevorzugter ≥ 80%, und am meisten bevorzugt ≥ 90% der feinen Mikropartikel, bezogen auf die Gesamtanzahl der feinen Mikropartikel des Wasch- und Reinigungsmittel, zu Beginn der Auflösungsphase in der Wasch- und/oder Reinigungsflotte freigesetzt werden.

In einer weiteren bevorzugten Ausführungsform weisen die Wasch- und Reinigungsmittel, feine Mikropartikel auf, die einem mechanischen Verdichtungsprozeß unterworfen wurden, wobei ≥ 10%, vorzugsweise ≥ 30%, bevorzugt ≥ 50%, weiter bevorzugt ≥ 70%, noch bevorzugter ≥ 80%, und am meisten bevorzugt ≥ 90% der feinen Mikropartikel, bezogen auf die Gesamtanzahl der feinen Mikropartikel des Wasch- und Reinigungsmittel, zu Beginn der Auflösungsphase mit einer Korngrößenverteilung von ≤ 50 µm in die Wasch- und/oder Reinigungsflotte freigesetzt werden.

Die feinen Mikropartikel können einem Verdichtungsprozeß unterworfen werden. Die Verdichtung der feinen Mikropartikel wird vorzugsweise so gewählt, daß bei der Freisetzung der feinen Mikropartikel in der Wasch- und/oder Reinigungsflotte diese unzerstört oder teilweise unzerstört freigesetzt werden. Mit der Formulierung unzerstört ist gemeint, daß die feinen Mikropartikel mit einer Korngrößenverteilung von ≤ 50 µm in die Wasch- und/oder Reinigungsflotte freigesetzt werden.

Erfindungsgemäß weisen die feinen Mikropartikel Wasch- und Reinigungsmittelbestandteile auf, die an Trägerstoffe absorbiert, adsorbiert und/oder in Hüllsubstanzen eingebettet sein können.

Die Trägerstoffe und/oder Hüllsubstanzen können ausgewählt werden aus der Gruppe, umfassend Salze wie Sulfate, Carbonate, Phosphate, Acetate; organische und anorganische Säuren, wie Citronensäure, Weinsäure, Apfelsäure, Alginsäure, Glutaminsäure; Zucker, wie Saccharose, Glucose, Fructose, Sorbitol, Lactose; Stärke- und Celluloseverbindungen, wie Kartoffelstärke, Maisstärke, Reisstärke, Maniokstärke, Johannesbrotkernmehl, Cyclodextrine, Algin, Gelatine; weitere natürliche und synthetische Polymere, wie Polyvinylpyrolidon (PVP), Polyacrylsäure, Polyacrylate, Maleinsäure-Acrylsäure-Copolymere, PEG; Silicate, wie Wasserglas, Zeolite, Metasilikate, Sodasilikate; Kieselsäuren; Tenside, wie Alkylbenzosulfonate, Fettalkoholsulfat, Stearate und/oder Harnstoff.

Die feinen Mikropartikel in Kombination mit Träger und/oder Hüllsubstanzen bilden Formkörper aus.

Die Wasch- und Reinigungsmittelbestandteile können als Gel, in fester und/oder flüssiger Form in den erfindungsgemäßen feinen Mikropartikeln enthalten sein.

Weitere Wasch- und Reinigungsmittelbestandteile der feinen Mikropartikel sind vorzugsweise ausgewählt aus der Gruppe umfassend Farbstoffe, Enzyme, Enzymstabilisatoren, Gerüststoffe, Stoffe zur Einstellung des pH-Wertes, Bleichmittel, Bleichaktivatoren, Silberschutzmittel, schmutzabweisende Substanzen, optische Aufheller, Vergrauungsinhibitoren, Desintegrationshilfsmittel, üblichen Inhaltsstoffen und/oder Mischungen davon.

Üblicherweise enthält das Wasch- und Reinigungsmittel die feinen Mikropartikel mit einem Gehalt von zwischen 10 Gew.-% und 70 Gew.-%, weiter bevorzugt von zwischen 10 Gew.% und 60 Gew.-%, noch bevorzugter von zwischen 10 Gew.-% und 50 Gew.-%, und am meisten bevorzugt von zwischen 20 Gew.-% und 40 Gew.-%, bezogen auf die Gesamtzusammensetzung des feine Mikropartikel aufweisenden Wasch- und Reinigungsmittels.

Besonders bevorzugt enthält das Wasch- und Reinigungsmittel die feinen Mikropartikel mit einem Gehalt von zwischen > 0,05 Gew.-% und 15 Gew.-% , vorzugsweise von zwischen 0, 1 Gew.-% und 10 Gew.%, bevorzugt von zwischen 0,2 Gew.% und 5 Gew.%, weiter bevorzugt von zwischen 0,3 Gew.-% und 3 Gew.-%, noch bevorzugter von zwischen 0,4 Gew.-% und 2 Gew.-%, und am meisten bevorzugt von zwischen 0,5 Gew.-% und 1 Gew.-%, bezogen auf die Gesamtzusammensetzung des feine Mikropartikel aufweisenden Wasch- und Reinigungsmittels.

Vorteilhaft ist es, daß das Mikropartikel einen Gehalt an wenigstens einem Wasch- und Reinigungsmittelbestandteil aus der Gruppe umfassend Tenside und Duftstoffe von zwischen 10 Gew.-% und 70 Gew.-%, weiter bevorzugt von zwischen 10 Gew.% und 60 Gew.-%, noch bevorzugter von zwischen 20 Gew.-% und 50 Gew.-%, und am meisten bevorzugt von zwischen 30 Gew.-% und 40 Gew.-%, bezogen auf die Gesamtzusammensetzung des feinen Mikropartikel, aufweist.

Besonders bevorzugt ist es, wenn das Wasch- und Reinigungsmittel feine Mikropartikel aufweist, wobei die Mikropartikel eine gleiche oder unterschiedliche Zusammensetzung von Wasch- und Reinigungsmittelbestandteilen aufweisen.

Die feine Mikropartikel enthaltenden erfindungsgemäßen Wasch- und Reinigungsmittel können in fester oder flüssiger Form vorliegen. Vorzugsweise weist das Wasch- und Reinigungsmittel mehrere Phasen auf. Noch bevorzugter ist das Wasch- und Reinigungsmittel eine temporäre Emulsion.

Das erfindungsgemäße Wasch- und Reinigungsmittel kann zur Reinigung harter Oberflächen und/oder weicher Oberflächen verwendet werden.

Die Wasch- und Reinigungsmittel können insbesondere als Geschirrspülmittel, Allzweckreiniger, Badreiniger, Fußbodenreiniger, Autoreiniger, Glasreiniger, Möbelpflegemittel bzw. Reiniger, Fassadenreiniger, Waschmittel und/oder dergleichen verwendet werden.

Weiter ist das Wasch- und Reinigungsmittel zur Reinigung von Haaren, Fasern, Textilien, Teppichen, Bekleidungsstücken, Lebensmitteln und/oder dergleichen geeignet.

Soweit nicht anders angegeben beziehen sich Gewichtsangaben auf die Gesamtzusammensetzung des feine Mikropartikel aufweisenden Wasch- und Reinigungsmittels.

Der Begriff Reinigungsmittelbestandteil im Sinne dieser Erfindung steht für Reinigungsmittelbestandteile und Waschmittelbestandteile, wenn nicht anders angegeben.

Erfindungsgemäß können alle im Stand der Technik bekannten Wasch- und Reinigungsmittel verwendet werden, soweit diese feine Mikropartikel enthalten können.

Es versteht sich von selbst, daß die feinen Mikropartikel die zur Herstellung der Mikropartikel verwendeten Reinigungsbestandteile aufweisen.

Die feinen Mikropartikel können einen und/oder mehrere Wasch- und Reinigungsmittelbestandteile aufweisen. Vorzugsweise weisen die feinen Mikropartikel einen Aktivgehalt von einem oder mehreren Wasch- und/oder Reinigungsmittelbestandteilen auf.

Die Wasch- und Reinigungsmittel können Mischungen von in ihrer Zusammensetzung gleichen oder unterschiedlichen, feinen Mikropartikeln mit einem und/oder mehreren Wasch- und Reinigungsmittelbestandteilen aufweisen.

Besonders bevorzugt ist es, wenn die feinen Mikropartikel im Wasch- und Reinigungsmittel homogen verteilt sind.

Nachfolgend werden die geeigneten Wasch- und Reinigungsmittelbestandteile im einzelnen näher erläutert.

### Gerüststoffe

Die erfindungsgemäßen feinen Mikropartikel der Wasch- und Reinigungsmittel können als Gerüststoff bzw. Builder alle üblicherweise in Wasch- und Reinigungsmitteln, insbesondere in Waschmitteln, eingesetzten Gerüststoffe enthalten, insbesondere also Zeolithe, Silikate, Carbonate, organische Cobuilder und auch die Phosphate.

Geeignete kristalline, schichtförmige Natriumsilikate besitzen die allgemeine Formel NaMSiₓO₂ₓ₊₁ H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl β- als auch δ-Natriumdisilikate Na₂Si₂O₅ yH₂O bevorzugt.

Einsetzbar sind auch amorphe Natriumsilikate mit einem Modul Na₂O: SiO₂ von 1:2 bis 1:3,3, vorzugsweise von 1:2 bis 1:2,8 und insbesondere von 1:2 bis 1:2,6. Insbesondere bevorzugt sind amorphe Silikate.

Ein verwendbarer feinkristalliner, synthetischer und gebundenes Wasser enthaltende Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O (1-n)K₂O·Al₂O₃·(2 - 2,5)SiO₂ · (3,5 - 5,5) H₂O

Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Als organische Cobuilder können in den erfindungsgemäßen feinen Mikropartikel der Wasch- und Reinigungsmitteln insbesondere Polycarboxylate / Polycarbonsäuren, polymere Polycarboxylate, Asparaginsäure, Polyacetale, Dextrine, weitere organische Cobuilder (siehe unten) sowie Phosphonate enthalten sein. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- und/oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Der Gehalt der feinen Mikropartikel der Wasch- und Reinigungsmittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die feinen Mikropartikel auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Cobuilder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C₆ des Saccharidrings oxidiertes Produkt kann besonders vorteilhaft sein.

Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP-A-0 232 202, EP-A-0 427 349, EP-A-0 472 042 und EP-A-0 542 496 sowie den internationalen Patentanmeldungen WO-A-92/18542, WO-A-93/08251, WO-A-94/28030, WO-A-95/07303, WO-A-95/12619 und WO-A-95/20608 bekannt. Ein an C6 des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4 524 009, US 4 639 325, in der europäischen Patentanmeldung EP-A-0 150 930 und der japanischen Patentanmeldung JP 93/339896 beschrieben werden.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Cobuilder werden beispielsweise in der internationalen Patentanmeldung WO-A-95/20029 beschrieben.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Wasch- und Reinigungsmittel auch Bleiche enthalten, bevorzugt sein Aminoalkanphosphonate, insbesondere DTPMP, in den feinen Mikropartikeln einzusetzen, oder Mischungen aus den genannten Phosphonaten zur Herstellung der feinen Mikropartikel zu verwenden.

Darüber hinaus können alle Verbindungen, die in der Lage sind, Komplexe mit Erdalkaliionen auszubilden, als Cobuilder eingesetzt werden.

Geeignete polymere Polycarboxylate zur Herstellung der feinen Mikropartikel sind beispielsweise die Natriumsalze der Polyacrylsäure oder der Polymethacrylsäure, beispielsweise solche mit einer relativen Molekülmasse von 800 bis 150000 (auf Säure bezogen). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5000 bis 200000, vorzugsweise 10000 bis 120000 und insbesondere 50000 bis 100000.

Der Gehalt der feine Mikropartikel enthaltenden Wasch- und/oder Reinigungsmittel an (co-)polymeren Polycarboxylaten liegt im üblichen Rahmen und beträgt vorzugsweise 1 bis 10 Gew.-%, bezogen auf die Gesamtzusammensetzung des Wasch- und Reinigungsmittels.

Insbesondere bevorzugt zur Herstellung der feinen Mikropartikel sind auch biologisch abbaubare Polymere aus mehr als zwei verschiedenen Monomereinheiten, beispielsweise solche, die gemäß der DE-A-43 00 772 als Monomere Salze der Acrylsäure und der Maleinsäure sowie Vinylalkohol bzw. Vinylalkohol-Derivate oder gemäß der DE-C-42 21 381 als Monomere Salze der Acrylsäure und der 2-Alkylallylsulfonsäure sowie Zucker-Derivate enthalten.

Weitere bevorzugte Copolymere zur Herstellung der feinen Mikropartikel sind solche, die in den deutschen Patentanmeldungen DE-A-43 03 320 und DE-A-44 17 734 beschrieben werden und als Monomere vorzugsweise Acrolein und Acrylsäure/Acrylsäuresalze bzw. Acrolein und Vinylacetat aufweisen.

Weitere geeignete Buildersubstanzen zur Herstellung der feinen Mikropartikel sind Oxidationsprodukte von carboxylgruppenhaltigen Polyglucosanen und/oder deren wasserlöslichen Salzen, wie sie beispielsweise in der internationalen Patentanmeldung WO-A-93/08251 beschrieben werden oder deren Herstellung beispielsweise in der internationalen Patentanmeldung WO-A-93/16110 beschrieben wird. Ebenfalls geeignet sind auch oxidierte Oligosaccharide gemäß der deutschen Patentanmeldung DE-A-196 00 018.

Ebenso zur Herstellung der feinen Mikropartikel sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, von denen in der deutschen Patentanmeldung DE-A-195 40 086 offenbart wird, daß sie neben Cobuilder-Eigenschaften auch eine bleichstabilisierende Wirkung aufweisen.

Weitere zur Herstellung der feinen Mikropartikel geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, beispielsweise wie in der europäischen Patentanmeldung EP-A-0 280 223 beschrieben, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Zusätzlich können die feinen Mikropartikel auch Komponenten enthalten, welche die Öl- und Fettauswaschbarkeit aus Textilien positiv beeinflussen. Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxy-propylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

### Inhaltsstoffe

Weitere geeignete Inhaltsstoffe der feinen Mikropartikel sind wasserlösliche anorganische Salze wie Bicarbonate, Carbonate, amorphe Silikate wie die oben erwähnten löseverzögerten Silikate oder Mischungen aus diesen; insbesondere werden Alkalicarbonat und amorphes Alkalisilikat, vor allem Natriumsilikat mit einem molaren Verhältnis Na2O : SiO2 von 1:1 bis 1:4,5, vorzugsweise von 1:2 bis 1:3,5, eingesetzt. Der Gehalt der feine Mikropartikel enthaltenden Wasch- und Reinigungsmittel an Natriumcarbonat beträgt dabei vorzugsweise bis zu 20 Gew.%, vorteilhafterweise zwischen 5 und 15 Gew.-%, bezogen auf die Gesamtzusammensetzung des Wasch- und Reinigungsmittel. Der Gehalt der feine Mikropartikel enthaltenden Wasch- und Reinigungsmittel an Natriumsilikat beträgt - falls es nicht als Buildersubstanz eingesetzt werden soll, im allgemeinen bis zu 10 Gew.-% und vorzugsweise zwischen 2 und 8 Gew.-%, ansonsten darüber, bezogen auf die Gesamtzusammensetzung des Wasch- und Reinigungsmittel.

Nach der Lehre der internationalen Patentanmeldung WO-A-94/01222 können Alkalicarbonate auch durch schwefelfreie, 2 bis 11 Kohlenstoffatome und gegebenenfalls eine weitere Carboxyl-und/oder Aminogruppe aufweisende Aminosäuren und/oder durch deren Salze ersetzt werden. Im Rahmen dieser Erfindung ist es dabei möglich, daß ein teilweiser bis vollständiger Austausch der Alkalicarbonate durch Glycin bzw. Glycinat erfolgt.

### Sonstige Bestandteile

Zu den sonstigen Bestandteilen der feinen Mikropartikel die verwendbar sind zählen beispielsweise Vergrauungsinhibitoren (Schmutzträger), Schauminhibitoren, Bleichmittel und Bleichaktivatoren, optische Aufheller, Enzyme, textilweichmachende Stoffe, Farb- und Duft sowie Neutralsalze wie Sulfate und Chloride in Form ihrer Natrium- oder Kaliumsalze.

### Mittel zur Einstellung des pH-Wertes

Zur Herabsetzung des pH-Wertes von Wasch- und Reinigungsmitteln, insbesondere Waschmitteln, können die feinen Mikropartikel auch saure Salze oder leicht alkalische Salze aufweisen. Bevorzugt sind hierbei als Säuerungskomponente Bisulfate und/oder Bicarbonate oder die obengenannten organischen Polycarbonsäuren, die gleichzeitig auch als Buildersubstanzen eingesetzt werden können. Insbesondere bevorzugt ist der Einsatz von Citronensäure.

### Tenside

Als Tenside zur Herstellung der feinen Mikropartikel können vorzugsweise Anion-, Kation-, Ampho-und/oder Niotenside verwendet werden, wobei anionische Tenside und nichtionische Tenside bevorzugt sind. Wichtige Inhaltsstoffe der erfindungsgemäßen Mittel und Inhaltsstoffe, die in dem erfindungsgemäßen Verfahren eingesetzt werden, sind Tenside, insbesondere Aniontenside, die wenigstens in Mengen von 0,5 Gew.-% in den erfindungsgemäßen feine Mikropartikel umfassenden Wasch- und Reinigungsmitteln bzw. erfindungsgemäß hergestellten feine Mikropartikel umfassenden Wasch- und Reinigungsmitteln enthalten sein sollten. Hierzu zählen insbesondere Sulfonate und Sulfate, aber auch Seifen.

### Aniontenside

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet. Auch geeignet sind Sulfonierungsprodukte von ungesättigten Fettsäuren, beispielsweise Ölsäure, in geringen Mengen, vorzugsweise in Mengen nicht oberhalb etwa 2 bis 3 Gew.-%.

Insbesondere sind α-Sulfofettsäurealkylester bevorzugt, die eine Alkylkette mit nicht mehr als 4C-Atomen in der Estergruppe aufweisen, beispielsweise Methylester, Ethylester, Propylester und Butylester. Mit besonderem Vorteil werden die Methylester der α-Sulfofettsäuren (MES), aber auch deren verseifte Disalze eingesetzt.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Die erfindungsgemäßen Wasch- und Reinigungsmitteln enthalten die vorgenannten Schwefelsäuremonoesterderivate aufgrund ihres hohen Schaumverhaltens nur in relativ geringen Mengen, beispielsweise in Mengen von 1 bis 5 Gew.-%, eingesetzt.

Weitere geeignete Aniontenside zur Herstellung der feinen Mikropartikel sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside bzw. die Sarkosinate und hier vor allem Sarkosinate von höheren und gegebenenfalls einfach oder mehrfach ungesättigten Fettsäuren wie Oleylsarkosinat.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Tri-ethanolamin, in den feinen Mikropartikeln vorliegen. Vorzugsweise liegen die anionischen Tenside in Form ihrer Natrium- oder Kaliumsalze, insbesondere in Form der Natriumsalze vor.

Bei der Auswahl der anionischen Tenside, die in den erfindungsgemäßen feinen Mikropartikeln der Wasch- und Reinigungsmitteln zum Einsatz kommen, stehen der Formulierungsfreiheit keine einzuhaltenden Rahmenbedingungen im Weg. Bevorzugte Wasch- und Reinigungsmittel weisen jedoch einen Gehalt an Seife auf, der 0,2 Gew.-%, bezogen auf das Gesamtgewicht des Wasch- und Reinigungsmittels, übersteigt. Bevorzugt einzusetzende anionische Tenside sind dabei die Alkylbenzolsulfonate und Fettalkoholsulfate, wobei bevorzugte Wasch- und Reinigungsmittel 2 bis 20 Gew.-%, vorzugsweise 2,5 bis 15 Gew.-% und insbesondere 5 bis 10 Gew.-% Fettalkoholsulfat(e), jeweils bezogen auf das Wasch- und Reinigungsmittelgewicht, enthalten

Die anionischen Tenside sind in den erfindungsgemäßen feinen Partikeln vorzugsweise in Mengen von 1 bis 100 Gew.-%, vorzugsweise von 1 bis 30 Gew.-% und insbesondere in Mengen von 5 bis 25 Gew.-% enthalten.

Neben den anionischen Tensiden und den kationischen, zwitterionischen und amphoteren Tensiden, sind vor allem nichtionische Tenside bevorzugt.

### Niotenside

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol zur Herstellung der feinen Mikropartikel eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO zur Herstellung der feinen Mikropartikel eingesetzt werden. Beispiele hierfür sind Talgfettalkohol mit 14 EO, 25 EO, 30 EO oder 40 EO.

Eine weitere Klasse bevorzugt eingesetzter nichtionischer Tenside zur Herstellung der feinen Mikropartikel, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden. Als Niotenside sind C₁₂-C₁₈-Fettsäuremethylester mit durchschnittlich 3 bis 15 EO, insbesondere mit durchschnittlich 5 bis 12 EO bevorzugt, während als Bindemittel - wie oben beschrieben - vor allem höher ethoxylierte Fettsäuremethylester vorteilhaft sind. Insbesondere C₁₂-C₁₈-Fettsäuremethylester mit 10 bis 12 EO können als Tenside eingesetzt werden.

Eine weitere Klasse von nichtionischen Tensiden, die vorteilhaft zur Herstellung der feinen Mikropartikel eingesetzt werden kann, sind die Alkylpolyglycoside (APG). Einsetzbare Alkypolyglycoside genügen der allgemeinen Formel RO(G)_{z}. in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Bevorzugt zur Herstellung der feinen Mikropartikel eingesetzt werden lineare Alkylpolyglucoside, also Alkylpolyglycoside, in denen der Polyglycosylrest ein Glucoserest und der Alkylrest ein n-Alkylrest ist.

Die erfindungsgemäßen feinen Mikropartikel können bevorzugt Alkylpolyglycoside enthalten, wobei Gehalte der Wasch- und Reinigungsmittel an APG über 0,2 Gew.-%, bezogen auf das gesamte Wasch- und Reinigungsmittel, bevorzugt sind. Besonders bevorzugte feine Mikropartikel enthaltende Wasch- und Reinigungsmittel weisen APG in Mengen von 0,2 bis 10 Gew.-%, vorzugsweise 0,2 bis 5 Gew.-% und insbesondere von 0,5 bis 3 Gew.-%.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können zur Herstellung der feinen Mikropartikel geeignet sein. Die Menge dieser nichtionischen Tenside beträgt vorzugsweise nicht mehr als die der ethoxylierten Fettalkohole, insbesondere nicht mehr als die Hälfte davon.

Als weitere Tenside kommen zur Herstellung der feinen Mikropartikel sogenannte Gemini-Tenside in Betracht. Hierunter werden im allgemeinen solche Verbindungen verstanden, die zwei hydrophile Gruppen und zwei hydrophobe Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, daß die hydrophilen Gruppen einen ausreichenden Abstand haben, damit sie unabhängig voneinander agieren können. Derartige Tenside zeichnen sich im allgemeinen durch eine ungewöhnlich geringe kritische Micellkonzentration und die Fähigkeit, die Oberflächenspannung des Wassers stark zu reduzieren, aus. In Ausnahmefällen werden jedoch unter dem Ausdruck Gemini-Tenside nicht nur dimere, sondern auch trimere Tenside verstanden.

Geeignete Gemini-Tenside zur Herstellung der feinen Mikropartikel sind beispielsweise sulfatierte Hydroxymischether gemäß der deutschen Patentanmeldung DE-A-43 21 022 oder Dimeralkoholbis- und Trimeralkohol-tris-sulfate und -ethersulfate gemäß der deutschen Patentanmeldung DE-A-195 03 061. Endgruppenverschlossene dimere und trimere Mischether gemäß der deutschen Patentanmeldung DE-A-195 13 391 zeichnen sich insbesondere durch ihre Bi- und Multifunktionalität aus. So besitzen die genannten endgruppenverschlossenen Tenside gute Netzeigenschaften und sind dabei schaumarm, so daß sie sich insbesondere für den Einsatz in maschinellen Wasch- oder Reinigungsverfahren eignen.

Eingesetzt werden können aber auch zur Herstellung der feinen Mikropartikel Gemini-Polyhydroxyfettsäureamide oder Poly-Polyhydroxyfettsäureamide, wie sie in den internationalen Patentanmeldungen WO-A-95/19953, WO-A-95/19954 und WO95-A-/19955 beschrieben werden.

Weitere geeignete Tenside zur Herstellung der feinen Mikropartikel sind Polyhydroxyfettsäureamide der Formel (I), 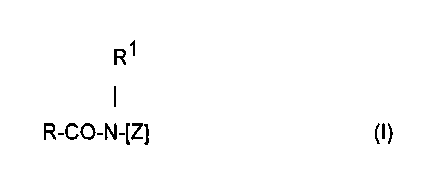 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (II), 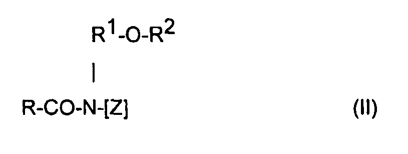 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielsweise durch Umsetzung mit Fettsäuremethylestern in Gegenwart eines Alkoxids als Katalysator in die gewünschten Polyhydroxyfettsäureamide überführt werden.

Bei der Herstellung der feinen Mikropartikel kommen als Tenside prinzipiell ebenfalls alle Tenside in Frage. Bevorzugt sind für diesen Anwendungszweck aber die vorstehend beschriebenen nichtionischen Tenside und hier vor allem die schwachschäumenden nichtionischen Tenside. Besonders bevorzugt sind die alkoxylierten Alkohole, besonders die ethoxylierten und/oder propoxylierten Alkohole. Dabei versteht der Fachmann allgemein unter alkoxylierten Alkoholen die Reaktionsprodukte von Alkylenoxid, bevorzugt Ethylenoxid, mit Alkoholen, bevorzugt im Sinne der vorliegenden Erfindung die längerkettigen Alkohole (C₁₀ bis C₁₈, bevorzugt zwischen C₁₂ und C₁₆, wie z. B. C₁₁-, C₁₂-, C₁₃-, C₁₄-, C₁₅-, C₁₆-,C₁₇- und C₁₈-Alkohole). In der Regel entstehen aus n Molen Ethylenoxid und einem Mol Alkohol, abhängig von den Reaktionsbedingungen ein komplexes Gemisch von Additionsprodukten unterschiedlichen Ethoxylierungsgrades. Eine weitere Ausführungsform besteht im Einsatz von Gemischen der Alkylenoxide bevorzugt des Gemisches von Ethylenoxid und Propylenoxid. Auch kann man gewünschtenfalls durch eine abschließende Veretherung mit kurzkettigen Alkylgruppen, wie bevorzugt der Butylgruppe, zur Substanzklasse der "verschlossenen" Alkoholethoxylaten gelangen, die ebenfalls im Sinne der Erfindung eingesetzt werden kann. Ganz besonders bevorzugt im Sinne der vorliegenden Erfindung sind dabei hochethoxylierte Fettalkohole oder deren Gemische mit endgruppenverschlossenen Fettalkoholethoxylaten.

### Bleichmittel

Die feinen Mikropartikel können auch Bleichmittel aufweisen.Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure.

Der Gehalt der feine Mikropartikel enthaltenden Wasch- und Reinigungsmitteln an Bleichmitteln beträgt vorzugsweise 5 bis 25 Gew.-% und insbesondere 10 bis 20 Gew.-%, bezogen auf die Gesamtzusammensetzung des Wasch- und Reinigungsmittel, wobei vorteilhafterweise Perboratmonohydrat oder Percarbonat eingesetzt wird.

Auch beim Einsatz der Bleichmittel ist es möglich, auf den Einsatz von Tensiden und/oder Gerüststoffen zu verzichten, so daß die Herstellung reiner, feiner Mikropartikel möglich ist. Sollen solche Bleichmittel zur Textilwäsche eingesetzt werden, ist der Einsatz von Natriumpercarbonat bevorzugt, unabhängig davon, welche weiteren Inhaltsstoffe in den Wasch- und Reinigungsmitteln enthalten sind.

Es können auch Bleichmittel aus der Gruppe der organischen Bleichmittel zur Herstellung der feinen Mikropartikel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesium-monoperphthalat, (b) die aliphatischen oder substituiert aliphatischen Peroxysäuren, wie Peroxylaurinsäure, Peroxystearinsäure, ε-Phthalimido-peroxycapronsäure [Phthaloiminoperoxyhexansäure (PAP)], o-Carboxybenzamido-peroxycapronsäure, N-nonenylamidoperadipinsäure und N-nonenylamidopersuccinate, und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxy-phthalsäuren, 2-Decyldiperoxybutan-1,4-disäure, N,N-Terephthaloyl-di(6-aminopercapronsäue) können zur Herstellung der feinen Mikropartikel eingesetzt werden.

Als Bleichmittel in den feinen Mikropartikeln können auch Chlor oder Brom freisetzende Substanzen eingesetzt werden. Unter den geeigneten Chlor oder Brom freisetzenden Materialien kommen beispielsweise heterocyclische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

### Bleichaktivatoren

Um beim Waschen oder Reinigen bei Temperaturen von 60 °C und darunter eine verbesserte Bleichwirkung zu erreichen, können Bleichaktivatoren in den feinen Mikropartikeln enthalten sein. Als Bleichaktivatoren zur Herstellung der feinen Mikropartikel können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.
Zusätzlich zu den konventionellen Bleichaktivatoren oder an deren Stelle können zur Herstellung der feinen Mikropartikel auch sogenannte Bleichkatalysatoren verwendet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu-und Ru-Amminkomplexe sind als Bleichkatalysatoren verwendbar.

Als Bleichaktivatoren können zur Herstellung der feinen Mikropartikel auch die aus den deutschen Patentanmeldungen DE-A-196 16 693 und DE-A-196 16 767 bekannten Enolester sowie acetyliertes Sorbitol und Mannitol beziehungsweise deren in der europäischen Patentanmeldung EP-A-0 525 239 beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, die aus den internationalen Patentanmeldungen WO-A-94/27970, WO-A-94/28102, WO-A-94/28103, WO-A-95/00626, WO-A-95/14759 und WO-A-95/17498 bekannt sind, verwendet werden. Die aus der deutschen Patentanmeldung DE-A-196 16 769 bekannten hydrophil substituierten Acylacetale und die in der deutschen Patentanmeldung DE-A-196 16 770 sowie der internationalen Patentanmeldung WO-A-95/14075 beschriebenen Acyllactame werden ebenfalls bevorzugt zur Herstellung der feinen Mikropartikel eingesetzt. Auch die aus der deutschen Patentanmeldung DE-A-44 43 177 bekannten Kombinationen konventioneller Bleichaktivatoren können zur Herstellung der feinen Mikropartikel eingesetzt werden. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf das gesamte feine Mikropartikel aufweisende Wasch- und Reinigungsmittel, enthalten.

Insbesondere beim Einsatz in maschinellen Waschverfahren kann es von Vorteil sein, das den feine Mikropartikel Mitteln übliche Schauminhibitoren zugesetzt sind. Als Schauminhibitoren eignen sich beispielsweise Seifen natürlicher oder synthetischer Herkunft, die einen hohen Anteil an C18-C24-Fettsäuren aufweisen. Geeignete nichttensidartige Schauminhibitoren sind beispielsweise Organopolysiloxane und deren Gemische mit mikrofeiner, ggf. silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere Silikon- und/oder Paraffin-haltige Schauminhibitoren, an eine granuläre, in Wasser lösliche bzw. dispergierbare Trägersubstanz gebunden. Insbesondere sind dabei Mischungen aus Paraffinen und Bistearylethylendiamiden bevorzugt.

Die feinen Mikropartikel können als Salze auch Polyphosphonsäuren aufweisen, vorzugsweise die neutral reagierenden Natriumsalze von beispielsweise 1-Hydroxyethan-1,1-diphosphonat, Diethylentriaminpenta-methylenphosphonat oder Ethylendiamintetramethylenphosphonat in Mengen von 0,1 bis 1,5 Gew.-%, bezogen auf die Gesamtzusammensetzung des feine Mikropartikel enthaltenden Wasch- und Reinigungsmittel.

### Enzyme

Als Enzyme kommen zur Herstellung der feinen Mikropartikel insbesondere solche aus der Klassen der Hydrolasen wie der Proteasen, Esterasen, Lipasen bzw. lipolytisch wirkende Enzyme, Amylasen, Glykosylhydrolasen und Gemische der genannten Enzyme in Frage. Alle diese Hydrolasen tragen zur Entfernung von Anschmutzungen wie protein-, fett- oder stärkehaltigen Verfleckungen bei. Zur Bleiche können auch Oxidoreduktasen eingesetzt werden. Besonders gut geeignet zur Herstellung der feinen Mikropartikel sind solche die aus Bakterienstämmen oder Pilzen wie Bacillus subtilis, Bacillus licheniformis, Streptomyceus griseus, Coprinus Cinereus und Humicola insolens sowie aus deren gentechnisch modifizierten Varianten gewonnene enzymatische Wirkstoffe. Vorzugsweise werden Proteasen vom Subtilisin-Typ und insbesondere Proteasen, die aus Bacillus lentus gewonnen werden. Dabei sind Enzymmischungen, beispielsweise aus Protease und Amylase oder Protease und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Auch Oxireduktasen sind geeignet.

Für die Herstellung der feinen Mikropartikel kommen neben den vorstehend genannten Enzymen zusätzlich noch Cellulasen in Betracht. Cellulasen und andere Glykosylhydrolasen können durch das Entfernen von Pilling und Mikrofibrillen zur Farberhaltung und zur Erhöhung der Weichheit des Textils beitragen. Als Cellulasen werden vorzugsweise Cellobiohydrolasen, Endoglucanasen und - Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert oder in Hüllsubstanzen eingebettet sein, um sie gegen vorzeitige Zersetzung zu schützen. Der Anteil der Enzyme oder Enzymmischungen kann beispielsweise etwa 0,1 bis 5 Gew.-%, vorzugsweise 0,5 bis etwa 4,5 Gew.-%, bezogen auf die feine Mikropartikel umfassende Wasch- und Reinigungsmittelzusammensetzung, betragen.

### Enzymstabilisatoren

Zusätzlich zu Phosphonaten können die feinen Mikropartikel noch weitere Enzymstabilisatoren enthalten. Beispielsweise können die Mikropartikel Natriumformiat enthalten. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Außer Calciumsalzen dienen auch Magnesiumsalze als Stabilisatoren. Besonders vorteilhaft ist jedoch der Einsatz von Borverbindungen, beispielsweise von Borsäure, Boroxid, Borax und anderen Alkalimetallboraten wie den Salzen der Orthoborsäure (H3BO3), der Metaborsäure (HBO2) und der Pyroborsäure (Tetraborsäure H2B4O7).

### Vergrauungsinhibitoren

Die feinen Mikropartikel können auch Vergrauungsinhibitoren enthalten. Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, sowie Polyvinylpyrrolidon beispielsweise in Mengen von 0,1 bis 5 Gew.-%, bezogen auf die Gesamtzusammensetzung des feine Mikropartikel enthaltenen Wasch- und Reinigungsmittel, eingesetzt.

### Silberschutzmittel

Erfindungsgemäß können zur Herstellung der feinen Mikropartikel, insbesondere für maschinelle Wasch- und Reinigungsmittel von Geschirr, zum Schutze des Spülgutes oder der Maschine Korrosionsinhibitoren verwendet werden, wobei Silberschutzmittel im Bereich des maschinellen Geschirrspülens eine besondere Bedeutung haben. Einsetzbar zur Herstellung der feinen Mikropartikel sind die bekannten Substanzen des Standes der Technik. Allgemein zur Herstellung der feinen Mikropartikel können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zur Herstellung der feinen Mikropartikel sind Benzotriazol und/oder Alkylaminotriazol zu verwenden. Man findet in Reinigerformulierungen darüber hinaus häufig aktivchlorhaltige Wasch- und Reinigungsmittel, die das Korrodieren der Silberoberfläche deutlich vermindern können, diese sind auch zur Herstellung der feinen Mikropartikel geeignet. Bei chlorfreien Reinigern werden zur Herstellung der feinen Mikropartikel besonders Sauerstoff- und stickstoffhaltige organische redoxaktive Verbindungen, wie zwei- und dreiwertige Phenole, z. B. Hydrochinon, Brenzkatechin, Hydroxyhydrochinon, Gallussäure, Phloroglucin, Pyrogallol bzw. Derivate dieser Verbindungsklassen verwendet. Auch salz- und komplexartige anorganische Verbindungen, wie Salze der Metalle Mn, Ti, Zr, Hf, V, Co und Ce können zur Herstellung der feinen Mikropartikel verwendet werden. Bevorzugt zur Herstellung der feinen Mikropartikel sind hierbei die Übergangsmetallsalze, die ausgewählt sind aus der Gruppe der Mangan und/oder Cobaltsalze und/oder -komplexe, besonders bevorzugt der Cobalt(ammin)-Komplexe, der Cobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut zur Herstellung der feinen Mikropartikel eingesetzt werden.

### Schmutzabweisende Substanzen

Zusätzlich können zur Herstellung der feinen Mikropartikel können auch schmutzabweisende Substanzen verwendet werden, welche die Öl- und Fettauswaschbarkeit aus Textilien positiv beeinflussen (sogenannte soil repellents). Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zur Herstellung der feinen Mikropartikel zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

### Optische Aufheller

Diese Stoffe, die auch "Weißtöner" genannt werden, werden zur Herstellung der feinen Mikropartikel eingesetzt, da sogar frisch gewaschene und gebleichte weißeWäsche einen leichten Gelbstich aufweist. Optische Aufheller sind organische Farbstoffe, die einen Teil der unsichtbaren UV-Strahlung des Sonnenlichts in längerwelliges blaues Licht umwandeln. Die Emission dieses blauen Lichts ergänzt die "Lücke" im vom Textil reflektierten Licht, so daß ein mit optischem Aufheller behandeltes Textil dem Auge weißer und heller erscheint. Da der Wirkungsmechanismus von Aufhellern deren Aufziehen auf die Fasern voraussetzt, unterscheidet man je nach "anzufärbenden" Fasern beispielsweise Aufheller für Baumwolle, Polyamid- oder Polyesterfasern. Die handelsüblichen für zur Herstellung der feinen Mikropartikel geeigneten Aufheller gehören dabei im wesentlichen fünf Strukturgruppen an Der Stilben-, der Diphenylstilben-, der Cumarin-Chinolin-, der Diphenylpyrazolingruppe und der Gruppe der Kombination von Benzoxazol oder Benzimidazol mit konjugierten Systemen. Ein Überblick über gängige Aufheller ist beispielsweise in G. Jakobi, A.Löhr "Detergents and Textile Washing", VCH-Verlag, Weinheim, 1987, Seiten 94 bis 100*,* zu finden. Geeignet sind z.B. Salze der 4,4'-Bis[(4-anilino-6-morpholino-s-triazin-2-yl)amino]-stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden.

### Duftstoffe

Duftstoffe können den zur Herstellung der erfindungsgemäßen feinen Mikropartikel zugesetzt werden, um den ästhetischen Eindruck der entstehenden Wasch- und Reinigungsmittel zu verbessern und dem Verbraucher neben der Reinigungsleistung und dem Farbeindruck ein sensorisch "typisches und unverwechselbares" Wasch- und Reinigungsmittel zur Verfügung zu stellen. Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe zur Herstellung der erfindungsgemäßen feinen Mikropartikel verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly- , Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die Duftstoffe können direkt in das feine Mikropartikel eingearbeitet werden, es kann aber auch vorteilhaft sein, die Duftstoffe auf die feinen Mikropartikel aufzubringen. Hierdurch wird eine verbesserte Haftung des Parfüms auf der Wäsche erreicht. Außerdem wird eine langsamere Duftfreisetzung für langanhaltenden Duft des Wasch- und Reinigungsmittel und der behandelten Textilien erreicht.

Als solche Trägermaterialien die zur Herstellung der erfindungsgemäßen feinen Mikropartikel geeignet sind, haben sich beispielsweise Cyclodextrine bewährt, wobei die Cyclodextrin-Parfüm-Komplexe zusätzlich noch mit weiteren Hilfsstoffen beschichtet werden können.

### Desintegrationshilfsmittel

Um den Zerfall der feinen Mikropartikel zu erleichtern, ist es möglich, Desintegrationshilfsmittel sogenannte Tablettensprengmittel, in diese einzuarbeiten, um die Zerfallszeiten zu verkürzen. Unter Tablettensprengmitteln bzw. Zerfallsbeschleunigern werden gemäß Römpp (9. Auflage, Bd. 6, S. 4440) und Voigt "Lehrbuch der pharmazeutischen Technologie" (6. Auflage, 1987, S. 182-184) Hilfsstoffe verstanden, die für den raschen Zerfall von Tabletten in Wasser oder Magensaft und für die Freisetzung der Pharmaka in resorbierbarer Form sorgen.

Diese Stoffe, die auch aufgrund ihrer Wirkung als "Spreng"mittel bezeichnet werden, vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen läßt. Altbekannte Desintegrationshilfsmittel sind beispielsweise Carbonat/Citronensäure-Systeme, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

Bevorzugte feine Mikropartikel aufweisende Wasch- und Reinigungsmittel enthalten 0,5 bis 10 Gew.-%, vorzugsweise 3 bis 7 Gew.-% und insbesondere 4 bis 6 Gew.-% eines oder mehrerer Desintegrationshilfsmittel, jeweils bezogen auf das Wasch- und Reinigungsmittelgewicht.

Als bevorzugte Desintegrationsmittel die zur Herstellung der erfindungsgemäßen feinen Mikropartikel geeignet sind, werden im Rahmen der vorliegenden Erfindung Desintegrationsmittel auf Cellulosebasis eingesetzt, so daß bevorzugte feine Mikropartikel aufweisende Wasch- und Reinigungsmittel ein solches Desintegrationsmittel auf Cellulosebasis in Mengen von 0,5 bis 10 Gew.-%, vorzugsweise 3 bis 7 Gew.-% und insbesondere 4 bis 6 Gew.-% enthalten. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose zur Herstellung der erfindungsgemäßen feinen Mikropartikel eingesetzt, die frei von Cellulosederivaten ist.

Als weiteres Desintegrationsmittel auf Cellulosebasis zur Herstellung der erfindungsgemäßen feinen Mikropartikel kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen.

### Farbstoffe

Um den ästhetischen Eindruck der erfindungsgemäßen Wasch- und Reinigungsmittel zu verbessern, können die feinen Mikropartikel aus Farbstoffen gebildet oder mit geeigneten Farbstoffen eingefärbt werden, wobei bevorzugt die aufhellerhaltige(n) Phase(n) die Gesamtmenge an Farbstoff(en) enthält/enthalten. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- und Reinigungsmittel und gegen Licht sowie keine ausgeprägte Substantivität gegenüber Textilfasern, um diese nicht anzufärben.

Bevorzugt für die Herstellung der erfindungsgemäßen feinen Mikropartikel sind alle Färbemittel, die im Waschprozeß oxidativ zerstört werden können sowie Mischungen derselben mit geeigneten blauen Farbstoffen, sog. Blautönern. Es hat sich als vorteilhaft erwiesen Färbemittel zur Herstellung der erfindungsgemäßen feinen Mikropartikel einzusetzen, die in Wasser oder bei Raumtemperatur in flüssigen organischen Substanzen löslich sind. Geeignet sind beispielsweise anionische Färbemittel, z.B. anionische Nitrosofarbstoffe. Ein mögliches Färbemittel ist beispielsweise Naphtholgrün (Colour Index (CI) Teil 1: Acid Green 1; Teil 2: 10020), das als Handelsprodukt beispielsweise als Basacid^{®} Grün 970 von der Fa. BASF, Ludwigshafen, erhältlich ist, sowie Mischungen dieser mit geeigneten blauen Farbstoffen. Als weitere Färbemittel kommen Pigmosol^{®} Blau 6900 (CI 74160), Pigmosol^{®} Grün 8730 (CI 74260), Basonyl^{®} Rot 545 FL (CI 45170), Sandolan^{®} Rhodamin EB400 (CI 45100), Basacid^{®} Gelb 094 (CI 47005), Sicovit^{®} Patentblau 85 E 131 (CI 42051), Acid Blue 183 (CAS 12217-22-0, CI Acidblue 183), Pigment Blue 15 (CI 74160), Supranol^{®} Blau GLW (CAS 12219-32-8, Cl Acidblue 221), Nylosan^{®} Gelb N-7GL SGR (CAS 61814-57-1, Cl Acidyellow 218) und/oder Sandolan^{®} Blau (CI Acid Blue 182, CAS 12219-26-0) zum Einsatz.

Bei der Wahl des Färbemittels muß beachtet werden, daß die Färbemittel keine zu starke Affinität gegenüber den textilen Oberflächen und hier insbesondere gegenüber Kunstfasern aufweisen. Gleichzeitig ist auch bei der Wahl geeigneter Färbemittel zu berücksichtigen, daß Färbemittel unterschiedliche Stabilitäten gegenüber der Oxidation aufweisen. Im allgemeinen gilt, daß wasserunlösliche Färbemittel gegen Oxidation stabiler sind als wasserlösliche Färbemittel. Abhängig von der Löslichkeit und damit auch von der Oxidationsempfindlichkeit variiert die Konzentration des Färbemittels in den Wasch- oder Reinigungsmitteln. Bei gut wasserlöslichen Färbemitteln, z.B. dem oben genannten Basacid^{®} Grün oder dem gleichfalls oben genannten Sandolan^{®} Blau, werden typischerweise Färbemittel-Konzentrationen im Bereich von einigen 10⁻² bis 10⁻³ Gew.-%, jeweils bezogen auf das gesamte Wasch- und Reinigungsmittel, gewählt. Bei den auf Grund ihrer Brillianz insbesondere bevorzugten, allerdings weniger gut wasserlöslichen Pigmentfarbstoffen, z.B. den oben genannten Pigmosol^{®}-Farbstoffen, liegt die geeignete Konzentration des Färbemittels in Wasch- oder Reinigungsmitteln dagegen typischerweise bei einigen 10⁻³ bis 10⁻⁴ Gew.-%, bezogen auf das gesamte Wasch- bzw Reinigungsmittel. Da die Farbstoffe vorzugsweise in einer Phase, d.h. einem kleineren Teilbereich der Formkörper eingesetzt werden, kann der Gehalt der Phase an Farbstoffen durchaus höher liegen.

Feine Mikropartikel lassen sich beispielsweise durch Mahlen herstellen, wobei in Luftstrahlmühlen Mikropartikelgrößen von unter 10 µm erhalten werden können. Für noch kleinere Mikropartikel, wie Nanopartikel, können die im Stand der Technik bekannten Verfahren verwendet werden.

Weitere geeignete Verfahren zur Herstellung der feinen Mikropartikel, insbesondere von Nanopartikeln, sind nachstehend aufgeführt:

### Kristallisationsverfahren

Bei diesem Verfahren handelt es sich um eine Kristallisation aus Lösungen mit großer Übersättigung, um so möglichst viele Kristallkeime zu erzeugen. Hierbei muß die Übersättigung möglichst im gesamten Medium, in dem die Kristallisation stattfindet, gleich hoch sein, damit praktisch an allen Stellen viele Keime für ein feinteiliges Kristallisat anfallen. Die Übersättigung aus Lösungen kann beispielsweise durch starke Abkühlung des Lösungsmittels, durch schlagartiges Verdampfen des Lösungsmittels, durch sehr schnelle Zugabe eines Antisolvents oder eines verdrängenden anderen Stoffes, z.B. Salzzugabe, durch Veränderung des pH-Wertes o.ä., je nach Lösungsverhalten des zu gewinnenden Stoffes, eingestellt werden. Geeignete Verfahren, deren Prinzip der Kristallisation auf einer hohen Übersättigung beruhen, umfassen Sprühprozesse, wie Sprühtrocknung, Versprühen in ein Antisolvent, siehe WO 90/03782 auf die im vollen Umfang bezug genommen wird, und Sprühprozesse mit gleichzeitiger Kühlung (Direct Contact Cooling).

### Erstarrung aus versprühter Schmelze

Bei diesem Verfahren werden die erfindungsgemäßen feinen Mikropartikel durch eine entsprechend feine Verdüsung der Schmelze erreicht, wobei die feinen Mikropartikel erst erstarren müssen bevor eine Reagglomeration stattfinden kann, z.B. Jet Priller von GMF Gouda.

Zum Beispiel mit dem PGSS-Prozess, siehe EP 0744992 auf die hier im vollem Umfang bezug genommen wird (?), ist eine Kühlung, Erstarrung und Lösungsmittelverdampfung in einem unter Erhalt der erfindungsgemäßen feinen Mikropartikel möglich.

### Fällungsreaktionen

Hierbei werden Reaktionen in Lösungen durchgeführt, wobei das Reaktionsprodukt im Gegensatz zu den Edukten im Lösungsmedium nicht oder nur gering löslich ist. Dadurch entsteht lokal eine sehr große Übersättigung, die zu entsprechend feinteiligem kristallisiertem Produkt führt. Dies ist auch als Sprühprozeß besonders effektiv - "Spray conversion Process".

Eine Erzeugung von erfindungsgemäßen feinen Mikropartikeln im Nanopartikelbereich kann durch reaktive Umsetzung auch in der Bulkphase durchgeführt werden, wobei häufig eine Feinverteilung eines Reaktionspartners vor der Reaktion durchgeführt wird - z.B.-Coazervation mit vorheriger Dispersion bei Mikroverkapselung; Feinvermahlung eines metallischen Precursors in einem Trägermedium bei der Herstellung von Keramikpulvern.

Je nach eingesetztem Waschmittelbestandteil, beispielsweise Duftstoff und/oder Tensid, ist das jeweils für die Herstellung der feinen Mikropartikel geeignete Verfahren auszuwählen.

Die feinen Tensid- und Parfüm-Mikropartikel können vorzugsweise in drei Angebotsformen eingesetzt werden:
- als Reinstoffpartikel
- als Mikrocompound auf einem ebenfalls feinteiligen Träger
- als feinste Mikropartikel (fest oder als Tropfen) in einer größeren Trägermatrix.

Die Beispiele 1 und 3 sind Vergleichsbeispiele. Hingegen betrifft das Beispiel 2 eine erfindungsgemäße Wasch- und Reinigungsmittel - Zusammensetzung mit feinen Mikropartikeln.

### Beispiel 1:

Waschmittel-Extrudat mit hohem Parfümölgehalt

### Rezeptur 1:

| | |
|---|---|
| Tensidgranulat | 75,00% |
| PEG 4000 | 4,00% |
| Zeolith A | 15,00% |
| Parfümöl | 6,00% |

**Tensidgranulat:**

| | |
|---|---|
| C ₉₋₁₃ Alkylbenzolsulfonat | 15,4 % |
| C ₁₂₋₁₈ Fettalkoholsulfat | 6,7% |
| Seife | 1,6% |
| Natriumcarbonat | 22,9% |
| Zeolith A | 45% |
| Na-Hydroxyethan-1,1-diphosphonat | 1,6% |
| Acrylsäure-Maleinsäure-Copolymer | 5,5% |
| Wasser, Salze | 1,3% |

Die festen Bestandteile werden in einem Mischer vorgemischt (Lödige-Pflugscharmischer), dabei wird das Parfümöl flüssig zugegeben.

Nach dem Mischen wurde das Vorgemisch in einem Doppelschneckenextruder zu Extrudaten (Durchmesser: 1,4 mm) verarbeitet.
Es ergab sich ein rieselfähiges Extrudat, dessen Auflösungsverhalten mit dem L-Test untersucht wurde.

### Rückstandsbestimmung (L-Test):

- 1000 ml Stadtwasser, werden bei 30°C in ein 2000 ml Becherglas geben;
- Einschalten des Rührers bei festgelegter Rührerdrehzahl;
- Einstreuen der Festgesetzte Menge des zu testenden Waschmittels und 90 Sekunden lösen (Stoppuhr);
- anschließend sofort die Waschlauge durch ein Sieb (Handwaschtest) abgießen;
- anschließend Trocknen der Siebe im Trockenschrank bei 40 °C bis zur Gewichtskonstanz ; und
- Auswiegen des Waschmittelrückstands.

### Beispiel 2:

Waschmittel-Extrudat mit hohem Parfümölgehalt

### Rezeptur 2:

| | |
|---|---|
| Tensidgranulat | 66,00% |
| PEG 4000 | 4,00% |
| Zeolith A | 15,00% |
| Parfümcompound | 15,00% |

Zusammensetzung des Tensidgranulats wie in Rezeptur 1.

Das Parfümcompound enthält 40% Parfümöl. In der Gesamtrezeptur ergibt sich somit ein Parfümölgehalt von 6%, wie in Rezeptur 1. Das Parfümcompound lag dabei als feines Mikropartikel, mit einer Korngröße x₉₀=25 µm, vor. Als Trägermaterial wurden Zeolith A, Acrylsäure-Maleinsäure-Copolymer und Cyclodextrin eingesetzt.

Alle Rezepturbestandteile wurden im Lödige-Mischer zu einem Vorgemisch verarbeitet, wobei das Parfümöl dem Vorgemisch nicht wie bei Rezeptur 1 als reine Flüssigkeit, sondern in Form feiner Mikropartikel zugegeben wurde. Die feinen Mikropartikel mit o.g. Korngröße enthielten dabei Trägersubstanz und Parfümöl, das Parfümöl lag somit im Vorgemisch in mikropartikulärer Form vor.
Die Weiterverarbeitung des Vorgemischs erfolgte wie bei Rezeptur 1. Nach der Extrusion wurden somit kompaktierte Formkörper enthaltend feine parfümölhaltige Mikropartikel erhalten.

Es ergab sich ein rieselfähiges Extrudat, dessen Auflösungsverhalten mit dem L-Test untersucht wurde.

### Beispiel 3:

Waschmittel-Extrudat mit hohem Parfümölgehalt

### Rezeptur 3:

| | |
|---|---|
| Tensidgranulat | 66,00% |
| PEG 4000 | 4,00% |
| Zeolith A | 15,00% |
| Compoundmaterial ohne Parfümöl | 9% |
| Parfümöl | 6,00% |

Die Zusammensetzung des Tensidgranulats ist dieselbe wie in Rezeptur 1.
Das Compoundmaterial entspricht dem Trägermaterial des Parfümcompounds aus der Rezeptur 2, allerdings ohne Parfümöl.

Parfümöl wurde, wie in Rezeptur 1, als Flüssigkeit dem Mischer zugegeben.
Die Weiterverarbeitung des Vorgemisches erfolgte wie bei Rezeptur 1.

Es ergab sich ein rieselfähiges Extrudat, dessen Auflösungsverhalten mit dem L-Test untersucht wurde.

### Vergleich der L-Test-Werte der Extrudate:

| | |
|---|---|
| | L-Test-Rückstand |
| Beispiel 1 | 25% |
| Extrudate nach Rezeptur 1 (Vergleich) | |
| Beispiel 2 | 12% |
| Extrudate nach Rezeptur 2 (erfindungsgemaß) | |
| Beispiel 3 | 27% |
| Extrudate nach Rezeptur 3 (Vergleich) | |

Wie aus den Werten zu ersehen ist, ergibt sich für Rezeptur 2 (Beispiel 2) ein deutlich günstigeres Auflösungs- bzw. Dispergierverhalten als für Rezeptur 1 und 3. Die Unterschiede ergeben sich dabei nicht aus den unterschiedlichen Rezepturanteilen (Rezeptur 2 gegen Rezeptur 1), sondern aus der Art der Rohstoffkonfektionierung des Parfümöls (Vergleich Rezeptur 2 - feine Mikropartikel - gegen Rezeptur 3). Der Austausch von Tensidgranulat gegen Trägercompound ohne Parfümöl (Rezeptur 3 gegen Rezeptur 1) hat im betrachteten Rahmen nur geringen Einfluß auf das Dispergierverhalten.

## Patentansprüche

1. Mechanisch verdichteter Wasch- und Reinigungsmittelformkörper in Form eines Granulats, Extrudats, Agglomerats und/oder einer Tablette, umfassend feine Mikropartikel mit Korngrößen ≤100 µm, welche 10 bis 90 Gew.-% eines oder mehrerer Wasch- und Reinigungsmittelbestandteile aus der Gruppe umfassend Tenside und Duftstoffe aufweisen, **dadurch gekennzeichnet, daß** die feinen Mikropartikel in Mengen von 10 Gew.-% bis 90 Gew.-% in dem Formkörper enthalten sind.

2. Wasch- und Reinigungsmittelformkörper nach Anspruch 1, **dadurch gekennzeichnet, daß** ≥ 10%, vorzugsweise ≥ 30%, bevorzugt ≥ 50%, weiter bevorzugt ≥ 70%, noch bevorzugter ≥ 80%, und am meisten bevorzugt ≥ 90% der feinen Mikropartikel, bezogen auf die Gesamtanzahl der feinen Mikropartikel, eine Korngrößenverteilung von ≤ 20 µm aufweisen.

3. Wasch- und Reinigungsmittelformkörper nach einem der Ansprüche 1 oder 2, **dadurch gekennzeichnet, daß** die feinen Mikropartikel eine Korngröße von zwischen 0,001 µm bis 50 µm, vorzugsweise von zwischen 0,01 µm bis 20 µm, bevorzugt von zwischen 0,05 µm bis 10 µm, weiter bevorzugt von zwischen 0,1 µm bis 5 µm, und am meisten bevorzugt von zwischen 0, 5 µm bis 1 µm, aufweisen.

4. Wasch- und Reinigungsmittelformkörper nach einem der Ansprüche 1 bis 3, **dadurch gekennzeichnet, daß** die feinen Mikropartikel Wasch- und Reinigungsmittelbestandteile aufweisen, die an Trägerstoffe absorbiert, adsorbiert und/oder in Hüllsubstanzen eingebettet sind.

5. Wasch- und Reinigungsmittelformkörper nach Anspruch 4, **dadurch gekennzeichnet, daß** die Trägerstoffe und/oder Hüllsubstanzen ausgewählt sind aus der Gruppe, umfassend Salze, organische und anorganische Säuren, Zucker, Stärke- und Celluloseverbindungen, weitere natürliche und synthetische Polymere, Silicate und Kieselsäuren, Tenside und/oder Harnstoff.

6. Wasch- und Reinigungsmittelformkörper nach einem der Ansprüche 1 bis 5, **dadurch gekennzeichnet, daß** die Wasch- und Reinigungsmittelbestandteile als Gel, in fester und/oder flüssiger Form in den feinen Mikropartikeln enthalten sind.

7. Wasch- und Reinigungsmittelformkörper nach einem der Ansprüche 1 bis 6, **dadurch gekennzeichnet, daß** der Wasch- und Reinigungsmittelformkörper die feinen Mikropartikel in Mengen von 10 Gew.-% bis 70 Gew.%, weiter bevorzugt von 10 Gew.-% bis 60 Gew.-%, noch bevorzugter von 10 Gew.-% bis 50 Gew.-%, und am meisten bevorzugt von 2.0 Gew.% bis 40 Gew.-%, bezogen auf die Gesamtzusammensetzung des feine Mikropartikel aufweisenden Wasch- und Reinigungsmittelformkörpers, enthält.

8. Wasch- und Reinigungsmittelformkörper nach einem der Ansprüche 1 bis 7, **dadurch gekennzeichnet, daß** das Mikropartikel einen Gehalt an wenigstens einem Wasch- und Reinigungsmittelbestandteil von zwischen zwischen 20 Gew.-% und 50 Gew.-%, und am meisten bevorzugt von zwischen 30 Gew.-% und 40 Gew.-%, bezogen auf die Gesamtzusammensetzung des feinen Mikropartikel, aufweist.

9. Wasch- und Reinigungsmittelformkörper nach einem der Ansprüche 1 bis 8, **dadurch gekennzeichnet, daß** die Mikropartikel eine gleiche oder unterschiedliche Zusammensetzung von Wasch- und Reinigungsmittelbestandteilen aufweisen.

10. Verwendung des Wasch- und Reinigungsmittelformkörpers nach einem der vorherigen Ansprüche zur Reinigung harter Oberflächen und/oder weicher Oberflächen.

11. Verwendung des Wasch- und Reinigungsmittelformkörpers nach einem der vorherigen Ansprüche als Geschirrspülmittel, Allzweckreiniger, Badreiniger, Fußbodenreiniger, Autoreiniger, Glasreiniger, Möbelpflegemittel, Möbelreiniger, Fassadenreiniger, Waschmittel und/oder dergleichen.

12. Verwendung des Wasch- und Reinigungsmittelformkörpers nach einem der vorherigen Ansprüche zur Reinigung von Haaren, Fasern, Textilien, Teppichen, Bekleidungsstücken, Lebensmitteln und/oder dergleichen.

13. Verfahren zur Herstellung von Wasch- und Reinigungsmittelformkörpern gemäß einem der Ansprüche 1 bis 9, **dadurch gekennzeichnet, daß** zur Bildung von Wasch- und Reinigungsmittelbestandteile aufweisenden Formkörpern die feinen Mikropartikel einem mechanischen Verdichtungsprozeß unterworfen werden.

## Claims

1. Mechanically compacted washing and cleaning agent shaped body in the form of a granulate, extrudate, agglomerate and/or a tablet, comprising fine microparticles with particle sizes of ≤ 100 µm, which have 10 to 90% by weight of one or more washing and cleaning agent components from the group consisting of surfactants and fragrances,
**characterized in that** the fine microparticles are present in amounts of from 10% by weight to 90% by weight in the shaped body.

2. Washing and cleaning agent shaped body according to Claim 1,
**characterized in that** ≥ 10%, preferably ≥ 30%, more preferably ≥ 50%, further preferably ≥ 70%, even more preferably ≥ 80%, and most preferably ≥ 90%, of the fine microparticles, based on the total number of fine microparticles, have a particle size distribution of ≤ 20 µm.

3. Washing and cleaning agent shaped body according to either Claim 1 or 2,
**characterized in that** the fine microparticles have a particle size from between 0.001 µm to 50 µm, preferably from between 0.01 µm to 20 µm, more preferably from between 0.05 µm to 10 µm, further preferably from between 0.1 µm to 5 µm, and most preferably from between 0.5 µm to 1 µm.

4. Washing and cleaning agent shaped body according to any of Claims 1 to 3,
**characterized in that** the fine microparticles have washing and cleaning agent components which are absorbed, adsorbed and/or embedded in coating substances on carrier materials.

5. Washing and cleaning agent shaped body according to Claim 4,
**characterized in that** the carrier materials and/or coating substances are chosen from the group consisting of salts, organic and inorganic acids, sugars, starch and cellulose compounds, further natural and synthetic polymers, silicates and silicas, surfactants and/or urea.

6. Washing and cleaning agent shaped body according to any of Claims 1 to 5,
**characterized in that** the washing and cleaning agent components are present in the fine microparticles as a gel, in solid and/or liquid form.

7. Washing and cleaning agent shaped body according to any of Claims 1 to 6,
**characterized in that** the washing and cleaning agent shaped body comprises the fine microparticles in amounts of from 10% by weight to 70% by weight, further preferably from 10% by weight to 60% by weight, still more preferably from 10% by weight to 50% by weight, and most preferably from 20% by weight to 40% by weight, based on the total composition of the washing and cleaning agent shaped body having fine microparticles.

8. Washing and cleaning agent shaped body according to any of Claims 1 to 7,
**characterized in that** the microparticle has a content of at least one washing and cleaning agent component of between 20% by weight and 50% by weight, and most preferably from between 30% by weight and 40% by weight, based on the total composition of the fine microparticle.

9. Washing and cleaning agent shaped body according to any of Claims 1 to 8,
**characterized in that** the microparticles have an identical or different composition of washing and cleaning agent components.

10. Use of the washing and cleaning agent shaped body according to any of the preceding claims for the cleaning of hard surfaces and/or soft surfaces.

11. Use of the washing and cleaning agent shaped body according to any of the preceding claims as dishwashing detergent, all-purpose cleaner, bath cleaner, floor cleaner, automobile cleaner, glass cleaner, furniture care compositions, furniture cleaners, façade cleaners, washing agents and/or the like.

12. Use of the washing and cleaning agent shaped body according to any of the preceding claims for the cleaning of hair, fibers, textiles, carpets, items of clothing, foods and/or the like.

13. Process for the preparation of washing and cleaning agent shaped bodies as in any of Claims 1 to 9,
**characterized in that** to form shaped bodies having washing and cleaning agent components, the fine microparticles are subjected to a mechanical compaction process.

## Revendications

1. Corps façonné d'agent de lavage et de nettoyage compacté mécaniquement, sous forme d'un granulat, d'un produit extrudé, d'un agglomérat et/ou d'un comprimé, comprenant des fines microparticules présentant des grosseurs de particule ≤ 100 µm, qui présentent 10 à 90% en poids d'un ou de plusieurs constituants d'agent de lavage et de nettoyage du groupe comprenant les agents tensioactifs et les parfums, **caractérisé en ce que** les fines microparticules sont contenues en des quantités de 10% en poids à 90% en poids dans le corps façonné.

2. Corps façonné d'agent de lavage et de nettoyage selon la revendication 1, **caractérisé en ce que** ≥ 10%, de préférence ≥ 30%, de préférence ≥ 50%, de manière particulièrement préférée ≥ 70%, de manière encore plus particulièrement préférée ≥ 80%, et de manière tout particulièrement préférée ≥ 90% des fines microparticules, par rapport au nombre total des fines microparticules présentent une répartition granulométrique ≤ 20 µm.

3. Corps façonné d'agent de lavage et de nettoyage selon l'une quelconque des revendications 1 ou 2, **caractérisé en ce que** les fines microparticules présentent une grosseur de 0,001 µm à 50 µm, de préférence de 0,01 µm à 20 µm, de préférence de 0,05 µm à 10 µm, de manière particulièrement préférée de 0,1 µm à 5 µm, et de manière tout particulièrement préférée de 0,5 µm à 1 µm.

4. Corps façonné d'agent de lavage et de nettoyage selon l'une quelconque des revendications 1 à 3, **caractérisé en ce que** les fines microparticules présentent des constituants d'agent de lavage et de nettoyage, qui sont absorbés, adsorbés sur des substances support et/ou enrobés dans des substances enveloppantes.

5. Corps façonné d'agent de lavage et de nettoyage selon la revendication 4, **caractérisé en ce que** les substances support et/ou les substances enveloppantes sont choisies dans le groupe comprenant les sels, les acides organiques et inorganiques, les sucres, les composés de type amidon et cellulose, d'autres polymères naturels et synthétiques, les silicates et les silices, les agents tensioactifs et/ou l'urée.

6. Corps façonné d'agent de lavage et de nettoyage selon l'une quelconque des revendications 1 à 5, **caractérisé en ce que** les constituants d'agent de lavage et de nettoyage sont contenus sous forme de gel, sous forme solide et/ou liquide dans les fines microparticules.

7. Corps façonné d'agent de lavage et de nettoyage selon l'une quelconque des revendications 1 à 6, **caractérisé en ce que** le corps façonné d'agent de lavage et de nettoyage contient les fines microparticules en des quantités de 10% en poids à 70% en poids, de préférence de 10% en poids à 60% en poids, de manière encore plus particulièrement préférée de 10% en poids à 50% en poids, et de manière tout particulièrement préférée de 20% en poids à 40% en poids, par rapport à la composition totale du corps façonné d'agent de lavage et de nettoyage présentant des fines microparticules.

8. Corps façonné d'agent de lavage et de nettoyage selon l'une quelconque des revendications 1 à 7, **caractérisé en ce que** la microparticule présente une teneur en au moins un constituant d'agent de lavage et de nettoyage entre 20% en poids et 50% en poids, de manière tout particulièrement préférée entre 30% en poids et 40% en poids, par rapport à la composition totale de la fine microparticule.

9. Corps façonné d'agent de lavage et de nettoyage selon l'une quelconque des revendications 1 à 8, **caractérisé en ce que** les microparticules présentent une composition identique ou différente de constituants d'agent de lavage et de nettoyage.

10. Utilisation du corps façonné d'agent de lavage et de nettoyage selon l'une quelconque des revendications précédentes pour le nettoyage de surfaces dures et/ou souples.

11. Utilisation du corps façonné d'agent de lavage et de nettoyage selon l'une quelconque des revendications précédentes, comme agent de lavage de vaisselle, agent de nettoyage universel, agent de nettoyage sanitaire, de sol, de voitures, de verre, agent d'entretien de meubles, agent de nettoyage de meubles, de façades, lessive et/ou analogues.

12. Utilisation du corps façonné d'agent de lavage et de nettoyage selon l'une quelconque des revendications précédentes pour le nettoyage de cheveux, de fibres, de textiles, de tapis, de vêtements, d'aliments et/ou analogues.

13. Procédé pour la production de corps façonnés d'agent de lavage et de nettoyage selon l'une quelconque des revendications 1 à 9, **caractérisé en ce que**, pour la formation des corps façonnés présentant les constituants d'agent de lavage et de nettoyage, les fines microparticules sont soumises à un processus de compactage mécanique.
